# EUROPEAN PATENT SPECIFICATION

(11) **EP 3 395 410 B1**
(45) Date of publication and mention of the grant of the patent: **15.12.2021**
(21) Application number: 17382219.8
(22) Date of filing: 25.04.2017
(51) Int. Cl.: A61Q 5/02, A61Q 5/12, A61Q 19/00, A61K 8/92

(54) **A COSMETIC COMPOSITION, METHOD AND PROCESS OF APPLICATION THEREOF**
EINE KOSMETISCHE ZUSAMMENSETZUNG, METHODE UND ANWENDUNGSVERFAHREN
COMPOSITION COSMÉTIQUE, SON PROCÉDÉ ET SON PROCESSUS D'APPLICATION

(43) Date of publication of application: 31.10.2018
(73) Proprietor: Revlon Consumer Products Corporation, New York, NY 10004 (US)
(72) Inventor: Beato Merino, Raquel, 08027 Barcelona (ES); Bermúdez Vico, Manuela, 08014 Barcelona (ES); Fernández Reyes, Sandra, 08014 Barcelona (ES)
(74) Representative: Gallardo, Antonio M.

(56) References cited:
- WO-A1-2007/033453
- WO-A1-2013/149323
- DE-A1-102009 029 197

## Description

### Technical Field

The present disclosure relates to a cosmetically effective combination of ingredients, a composition which contains this combination, a method and a process of treatment and/or care of the hair which comprises the topical application of the composition.

### Background

The cosmetic treatment of the hair is an element of the personal care. Different types of hair cosmetic compositions and hundreds of different ingredients are contained in the hair cosmetic compositions of the state of the art. For example, DE 10 2009 029197 A1 discloses cosmetic hair formulations comprising Inca Inchi oil.

There is the need in the state of the art of new combinations of ingredients which provide an improvement of at least one of the properties desired for a hair cosmetic product, for example; improvement of the feel, strength, elasticity, wet and/or dry combability, flash effect, shine or gloss, or hair repair.

### Summary

In a first aspect, the composition comprises a cosmetically effective amount of Murumuru butter that ranges from 0.001% to 5% by weight, a cosmetically effective amount of Acai berry extract that ranges from 0.0001% to 2% by weight and a cosmetically effective amount of Inca Inchi oil that ranges from 0.005% to 15% by weight of the total composition.

In another aspect, the method of cosmetic treatment and/or care of the hair comprises the topical application of the composition comprising a cosmetically effective amount of Murumuru butter that ranges from 0.001% to 5% by weight, a cosmetically effective amount of Acai berry extract that ranges from 0.0001% to 2% by weight and a cosmetically effective amount of Inca Inchi oil that ranges from 0.005% to 15% by weight of the total composition.

In a third aspect, the process of cosmetic treatment and/or care of the hair which comprises the steps: A) topical application of a cleansing composition to the hair, B) rinsing the cleansing composition of step A), C) topical application of a conditioner composition to the hair, D) optionally, the conditioner composition of step C) is rinsed-off, wherein at least one of the cleansing composition of step A) or the conditioner composition of step C) is a composition comprising a cosmetically effective amount of Murumuru butter that ranges from 0.001% to 5% by weight, a cosmetically effective amount of Acai berry extract that ranges from 0.0001% to 2% by weight and a cosmetically effective amount of Inca Inchi oil that ranges from 0.005% to 15% by weight of the total composition.

### Detailed Description

Embodiments of the present invention are discussed in detail below. In describing embodiments, specific terminology is employed for the sake of clarity. However, the invention is not intended to be limited to the specific terminology so selected. While specific exemplary embodiments are discussed, it should be understood that this is done for illustration purposes only. A person skilled in the relevant art will recognize that other components and configurations can be used without parting from the spirit and scope of the invention. While a number of embodiments and features are described herein, it is to be understood that the various features of the invention and aspects of embodiments, even if described separately, may be combined unless mutually exclusive or contrary to the specific description. All references cited herein are incorporated by reference as if each had been individually incorporated.

As used herein, the transitional term "comprising," which is synonymous with "including," "containing," or "characterized by," is inclusive or open-ended and does not exclude additional, un-recited elements or method steps. However, in each recitation of "comprising" herein, it is intended that the term also encompass, as alternative embodiments, the phrases "consisting essentially of" and "consisting of", where "consisting of" excludes any element or step not specified and "consisting essentially of" permits the inclusion of additional un-recited elements or steps that do not materially affect the essential or basic and novel characteristics of the composition or method under consideration.

The present disclosure relates to a composition comprising a cosmetically effective amount of Murumuru butter, Acai berry extract and Inca Inchi oil.

This composition improves the hair shine or gloss, as it is shown in the examples.

For purposes of this disclosure the term "care" refers to the maintenance of the qualities of the hair and includes the hair hygiene. These qualities are subject to improvement and maintained through a cosmetic treatment and/or care of the hair both in healthy subjects as well as those which present diseases and/or disorders of the scalp, such as and not restricted to, ulcers and lesions on the scalp, psoriasis, dermatitis, dandruff, or hair loss, among others. "Hair" means in this disclosure the human hair that grows on the scalp.

The ingredient "Murumuru butter" is the fat or butter obtained from the seed of Murumuru palm tree (*Astrocaryum murumuru*)*,* also known with the INCI name: Astrocaryum Murumuru Seed Butter.

The ingredient "Acai berry extract" is the extract of the fruit of *Euterpe oleracea* palm tree, commonly known as Acai palm, or Açaí palm, or Assai palm. This extract is also known with the INCI name: Euterpe Oleracea Fruit Extract.

The ingredient "Inca Inchi oil" is the oil obtained from the seed of *Plukenetia volubilis* plant, commonly known as Inchi, Inca Inchi, Sacha Inchi, Sacha peanut, Inca peanut or mountain peanut. This oil is also known with the INCI name: Plukenetia Volubilis Seed Oil.

"Cosmetically effective amount" is understood to mean a nontoxic but sufficient amount of the ingredients of the composition to provide the desired effect. The ingredients are used in the composition at cosmetically effective concentrations to achieve the desired effect.

The composition also may include, but is not limited to, tocopherol and/or maltodextrin. Tocopherol is an antioxidant that may be added to some marketed Inca Inchi oils. Tocopherol may be added to Inca Inchi oil in a ratio 0.1:99.9 to 4:96 of Tocopherol:Inca Inchi oil. Maltodextrin is a bulking agent that may be added to marketed Acai berry extract. Maltodextrin may be added to Acai berry extract in a ratio 30:70 to 10:90 of Maltodrextin:Acai berry extract. The composition that may include tocopherol and/or maltodextrin, may be a cosmetic composition, or a hair cosmetic composition, or a hair care composition.

The cosmetically effective amount of Murumuru butter in the composition may range from about 0.001% to about 5%, from about 0.005% to about 3%, or from about 0.01% to about 1% by weight of the total composition. This composition may be a cosmetic composition, or a hair cosmetic composition, or a hair care composition.

The cosmetically effective amount of Acai berry extract in the composition may range from about 0.0001% to about 2%, from about 0.0005% to about 1%, or from about 0.005% to about 0.1% by weight of the total composition. This composition may be a cosmetic composition, or a hair cosmetic composition, or a hair care composition.

The cosmetically effective amount of Inca Inchi oil in the composition may range from about 0.005% to about 15%, from about 0.005% to about 5%, or from about 0.01% to about 2.5% by weight of the total composition. This composition may be a cosmetic composition, or a hair cosmetic composition, or a hair care composition.

The cosmetically effective amount of Murumuru butter in the composition may range from about 0.001% to about 5%, the cosmetically effective amount of Acai berry extract in the composition may range from about 0.0001% to about 2% and the cosmetically effective amount of Inca Inchi oil in the composition may range from about 0.005% to about 15%. Maltodextrin may be added to Acai berry extract in a ratio 30:70 to 10:90 of Maltodrextin:Acai berry extract. Tocopherol may be added to Inca Inchi oil in a ratio 0.1:99.9 to 4:96 of Tocopherol:Inca Inchi oil. The composition may be a cosmetic composition, or a hair cosmetic composition, or a hair care composition.

The cosmetically effective amount of Murumuru butter in the composition may range from about 0.005% to about 3%, the cosmetically effective amount of Acai berry extract in the composition may range from about 0.0005% to about 1% and the cosmetically effective amount of Inca Inchi oil in the composition may range from about 0.005% to about 5%. Maltodextrin may be added to Acai berry extract in a ratio 30:70 to 10:90 of Maltodrextin:Acai berry extract. Tocopherol may be added to Inca Inchi oil in a ratio 0.1:99.9 to 4:96 of Tocopherol:lnca Inchi oil. The composition may be a cosmetic composition, or a hair cosmetic composition, or a hair care composition.

The cosmetically effective amount of Murumuru butter in the composition may range from about 0.01% to about 1%, the cosmetically effective amount of Acai berry extract in the composition may range from about 0.005% to about 0.1% and the cosmetically effective amount of Inca Inchi oil in the composition may range from about 0.01% to about 2.5%. Maltodextrin may be added to Acai berry extract in a ratio 30:70 to 10:90 of Maltodrextin:Acai berry extract. Tocopherol may be added to Inca Inchi oil in a ratio 0.1:99.9 to 4:96 of Tocopherol:lnca Inchi oil. The composition may be a cosmetic composition, or a hair cosmetic composition, or a hair care composition.

The composition may also comprise amino acids of keratin, obtained from the complete hydrolysis of keratin, and with INCI name: Keratin Amino Acids. The average of Keratin Amino Acids in the composition may range between about 0.001% and about 3%, between about 0.005% and about 1%, or between about 0.01% and about 0.5% by weight of the total composition. The composition that may comprise Keratin Amino Acids, may be a cosmetic composition, or a hair cosmetic composition, or a hair care composition.

The composition may be a cosmetic composition. The cosmetic composition may be a hair cosmetic composition. The hair cosmetic composition may be a hair care composition. The hair care composition may be a shampoo, an oil, a serum, a rinse-off hair conditioner, a rinse-off hairmask, a leave-on hair conditioner or a leave-on hair mask. The hair care composition may be a rinse-off hair conditioner a rinse-off hair mask, a leave-on hair conditioner or a leave-on hair mask. A "rinse off" conditioner or mask is a conditioner or mask washed off with water and not left on the hair after the application time. A "leave on" conditioner or mask is a conditioner or mask which is intended to stay in prolonged contact with the hair, generally, until the next cleaning of the hair.

The composition may be a cosmetic composition, or a hair cosmetic composition, or a hair care composition, and the composition may comprise at least one additional cosmetic ingredient selected from the group of humectants, emollients, emulsifiers, surfactants, viscosity increasing ingredients, thickeners, binders, bulking agents, solvents, emulsion stabilizers, hair conditioners, natural extracts, antioxidants, pH adjusters, hair fixatives, film formers, UV filters, opacifying agents, fragrance ingredients, preservatives and cosmetic colorants. "Humectants" are ingredients used in cosmetic products to retard moisture loss from the product during use. "Emollients" are cosmetic ingredients which help to maintain the soft, smooth, and pliable appearance of hair, and they act as lubricants. "Emulsifiers" are cosmetic ingredients required for the formation of emulsions that stabilize an emulsion by increasing its kinetic stability. "Surfactants" are cosmetic ingredients that reduce surface tension of water or reduce the interfacial tension between two immiscible substances. "Viscosity increasing agents" are cosmetic ingredients used to thicken cosmetic products. "Binders" are ingredients added to compounded dry powder mixtures of solids and the like to provide adhesive qualities during and after compression. "Bulking agents" are cosmetic ingredients used to increase the volume of a cosmetic. "Solvents" are liquids employed to dissolve components found useful in cosmetics. "Emulsion stabilizers" are cosmetic ingredients that assist in the formation and the stabilization of emulsions. Emulsion stabilizers do not act as primary emulsifiers, but prevent or reduce the coalescence of emulsified droplets, and they enhance the activity of emulsifiers. "Hair conditioners" are ingredients used to create special effects on hair, and they include ingredients which enhance the appearance and feel of hair, increase hair body or suppleness, facilitate styling, improve gloss or sheen and improve the texture of hair, including hair that has been damaged by chemical or physical action. "Natural extracts" are substances or active ingredients with desirable properties that are removed from a plant, flower, alga, fungus or bacteria, usually by treating it with a solvent, to be used for a particular purpose. "Antioxidants" are ingredients employed in cosmetics to prevent or retard product spoilage from rancidity or deterioration from reaction with oxygen, they play a vital role in maintaining the quality, integrity, and safety of cosmetic products. "pH adjusters" are acids, bases, or buffering agents which are used to control the pH of cosmetic products or ingredients. "Hair fixatives" are ingredients which impart holding or style-retention properties to hair. "Film formers" are ingredients which produce a continuous film on hair. "UV filters" are ingredients used to absorb or reflect the UV rays that are contained in sun light or in artificial light, they can be used to protect the skin from the harmful effects of UV light or to protect cosmetic products and ingredients. "Opacifying agents" are ingredients deliberately added to cosmetic products to reduce their clear or transparent appearance. "Fragrance ingredients" are any natural or synthetic substance or substances used to impart an odor to a cosmetic product. "Preservatives" are ingredients which prevent or retard microbial growth and thus protect cosmetic products from spoilage, they also protect the product from inadvertent contamination by the consumer during use. "Cosmetic colorants" are cosmetic ingredients which impart color to finished products.

The present disclosure also relates to a method of treatment and/or care of the hair which comprises the topical application of a composition comprising a cosmetically effective amount of Murumuru butter, Acai berry extract and Inca Inchi oil.

The method of treatment and/or care of the hair may be a method of cosmetic treatment and/or care of the hair and the composition may be a cosmetic composition. The method of treatment and/or care of the hair may be a cosmetic method of increasing the shine or gloss of the hair. By "increasing the shine or gloss of the hair" it is understood that the value of gloss measured at an angle of 8° or the ISO 2470 brightness value measured with an spectrophotometer for a hair lock after the topical application of the disclosed composition is higher than before the topical application of the disclosed composition which comprises a cosmetically effective amount of a Murumuru butter, an Acai berry extract and an Inca Inchi oil. The spectrophotometer may be a spectrophotometer CM-2600d (Konica Minolta) with the software Color data software CM-S100w Spectra Magic^{™} NX (Konica Minolta).

The composition in the disclosed method may be topically applied to the hair from about 20 seconds to about 45 minutes, from about 30 seconds to about 30 minutes, from about 1 minute to about 15 minutes. The composition may be a cosmetic composition. The cosmetic composition may be a hair cosmetic composition. The hair cosmetic composition may be a hair care composition. The hair care composition may be a shampoo, an oil, a serum, a rinse-off hair conditioner or a rinse-off hair mask. The hair care composition may be a rinse-off hair conditioner or a rinse-off hair mask.

The composition in the disclosed method may be left-on the hair for at least 2 hours, for at least 4 hours, for at least 8 hours, until the next hair wash. The composition may be a cosmetic composition. The cosmetic composition may be a hair cosmetic composition. The hair cosmetic composition may be a hair care composition. The hair care composition may be an oil, a serum, a leave-on hair conditioner or a leave-on hair mask. The hair care composition may be a leave-on hair conditioner or a leave-on hair mask.

The present disclosure also relates to a process of treatment and/or care of the hair which comprises the steps: A) topical application of a cleansing composition is to the hair, B) rinsing the cleansing composition of step A), C) topical application of a conditioner composition to the hair, D) optionally, the conditioner composition of step C) is rinsed-off, wherein at least one of the cleansing composition of step A) or the conditioner composition of step C) is a composition comprising a cosmetically effective amount of Murumuru butter, Acai berry extract and Inca Inchi oil.

The process of treatment and/or care of the hair may be a process of cosmetic treatment and/or care of the hair. The composition in the process of treatment and/or care of the hair comprising a cosmetically effective amount of Murumuru butter, Acai berry extract and Inca Inchi oil may be a cosmetic composition.

The composition in the process of treatment and/or care of the hair comprising a cosmetically effective amount of Murumuru butter, Acai berry extract and Inca Inchi oil may be the conditioner composition of step C). The conditioner composition of step C) may be a rinse-off hair conditioner, a rinse-off hair mask, a leave-on hair conditioner or a leave-on hair mask.

The composition in the process of treatment and/or care of the hair comprising a cosmetically effective amount of Murumuru butter, Acai berry extract and Inca Inchi oil may be the cleansing composition of step A). The cleansing composition of step A) may be a shampoo.

Except in the Examples, or where otherwise explicitly indicated, all numerical quantities in this description specifying amounts of materials, application conditions, and the like, are to be understood as approximated, i.e., subject to a variability of plus or minus 5 percent, more preferably of plus or minus 3 percent, more preferably of plus or minus 1 percent, more preferably of plus or minus 0.1 percent, even more preferably of plus or minus 0.01 percent over the indicated value. It is to be understood that the upper and lower amount, range, and ratio limits set forth herein may be independently combined. Similarly, the ranges and amounts for each element of the technology described herein can be used together with ranges or amounts for any of the other elements.

### Examples

The following examples are included for illustrative purposes only and should not be construed as limitations on the invention claimed herein.

### EXAMPLE 1

### Rinse-off hair mask

In a suitable vessel Water, Glycerin, Citric Acid, Steartrimonium Chloride, Isopropyl Alcohol, Dicetyldimonium Chloride, Lauryl Alcohol, Myristyl Alcohol and Cetearyl Alcohol are mixed at 80°C with stirring.

Then, a premix of Polyquaternium-28 with Water is added to the mixture at 80°C.

The temperature of the vessel is decreased to 40°C and Phenoxyethanol, Panthenol and Ethylhexylglycerin are added to the mixture with stirring.

Then, Murumuru butter and Inca Inchi oil are added one by one to the mixture at 40°C with stirring.

In the next step, a pre-blend of Glycerin, Acai berry extract, Maltodextrin and Water is added to the mixture at a temperature under 40°C. Finally, the perfume (Parfum, Hexyl Cinnamal, Limonene, Benzyl Salicylate) is added. Table 1 shows the average of each ingredient in the formula.

**Table 1**

| **INGREDIENT (INCI Name)** | **CONCENTRATION (%)** | |
|---|---|---|
| Aqua (Water (Eau)) | | q.s.p. 100 |
| Cetearyl Alcohol | | 7.50000 |
| Plukenetia Volubilis Seed Oil | | 1.99850 |
| Glycerin | | 1.50000 |
| Astrocaryum Murumuru Seed Butter | | 1.00000 |
| Dicetyldimonium Chloride | | 0.58277 |
| Lauryl Alcohol | | 0.54750 |
| Panthenol | | 0.49000 |
| Isopropyl Alcohol | | 0.36660 |
| Ethylhexylglycerin | | 0.30000 |
| Myristyl Alcohol | | 0.20250 |
| Polyquaternium-28 | | 0.20000 |
| Euterpe Oleracea Fruit Extract | | 0.03808 |
| Citric Acid | | 0.01600 |
| Maltodextrin | | 0.00875 |
| Parfum (Fragrance) | | 0.38140 |
| Hexyl Cinnamal | | 0.04751 |
| Limonene | | 0.03192 |
| Benzyl Salicylate | | 0.02401 |
| Steartrimonium Chloride | | 0.80000 |
| Phenoxyethanol | | 0.70000 |

| | | |
|---|---|---|
| *(q.s.p. 100: quantity sufficient for 100)* | | |

A person skilled in the art will easily recognizes that similar compositions to this composition of Example 1, can be prepared in the form of other rinse-off hair masks, leave-on hair conditioners, shampoos, or oil based shampoos, for example.

### EXAMPLE 2

### Gloss measurement for the composition of Example 1 vs comparative compositions

The gloss measurement is tested in 4 hair locks from yak, each lock treated with a different composition. The first lock is treated with the composition of Example 1 and the remaining three locks are treated with one of the three comparative compositions. The comparative compositions contain the ingredients at the averages of Example 1, but with only 1 of the 3 ingredients of the claimed composition in each comparative composition, see Table 2. That is, comparative composition 1 contains 0.03808% Acai berry extract and its bulking agent Maltodextrin 0.00875%, but not Inca Inchi oil, neither Murumuru butter; comparative composition 2 contains 1.0000% Murumuru butter, but not Inca Inchi oil, neither Acai berry extract, nor maltodextrin; and comparative composition 3 contains 1.99850% Inca Inchi oil, but not Murumuru butter, neither Acai berry extract, nor maltodextrin.

**Table 2**

| **INGREDIENT (INCI Name)** | **COMPARATIVE COMPOSITION 1, CONCENTRATION (%)** | | **COMPARATIVE COMPOSITION 2, CONCENTRATION (%)** | | **COMPARATIVE COMPOSITION 3, CONCENTRATION (%)** | |
|---|---|---|---|---|---|---|
| Aqua (Water (Eau)) | | q.s.p. 100 | | q.s.p. 100 | | q.s.p. 100 |
| Cetearyl Alcohol | | 7.50000 | | 7.50000 | | 7.50000 |
| Plukenetia Volubilis Seed Oil | | --- | | --- | | 1.99850 |
| Glycerin | | 1.50000 | | 1.50000 | | 1.50000 |
| Astrocaryum Murumuru Seed Butter | | --- | | 1.00000 | | --- |
| Dicetyldimonium Chloride | | 0.58277 | | 0.58277 | | 0.58277 |
| Lauryl Alcohol | | 0.54750 | | 0.54750 | | 0.54750 |
| Panthenol | | 0.49000 | | 0.49000 | | 0.49000 |
| Isopropyl Alcohol | | 0.36660 | | 0.36660 | | 0.36660 |
| Ethylhexylglycerin | | 0.30000 | | 0.30000 | | 0.30000 |
| Myristyl Alcohol | | 0.20250 | | 0.20250 | | 0.20250 |
| Polyquaternium-28 | | 0.20000 | | 0.20000 | | 0.20000 |
| Euterpe Oleracea Fruit Extract | | 0.03808 | | --- | | --- |
| Citric Acid | | 0.01600 | | 0.01600 | | 0.01600 |
| Maltodextrin | | 0.00875 | | --- | | --- |
| Parfum (Fragrance) | | 0.38140 | | 0.38140 | | 0.38140 |
| Hexyl Cinnamal | | 0.04751 | | 0.04751 | | 0.04751 |
| Limonene | | 0.03192 | | 0.03192 | | 0.03192 |
| Benzyl Salicylate | | 0.02401 | | 0.02401 | | 0.02401 |
| Steartrimonium Chloride | | 0.80000 | | 0.80000 | | 0.80000 |
| Phenoxyethanol | | 0.70000 | | 0.70000 | | 0.70000 |

| | | | | | | |
|---|---|---|---|---|---|---|
| *(q.s.p. 100: quantity sufficient for 100)* | | | | | | |

The gloss is measured for each dry and combed hair lock with a measurement angle of 8° and as ISO 2470 brightness with a spectrophotometer CM-2600d (Konica Minolta) with the software Color data software CM-S100w Spectra Magic^{™} NX (Konica Minolta). This gloss measurement is the pattern for each lock after treatment with the hereinabove disclosed compositions.

Each lock is plunged in warm water for 30 seconds, and the locks are uniformly wetted. Then, the composition of Example 1 or the comparative compositions are applied to the hair locks and the compositions are left on the hair locks for 5 minutes. Then, the hair locks are washed with plenty of warm water for 1 minute. The hair locks are hung and air-dried. The gloss is measured when the hair locks are dry and combed with the above mentioned equipment and software.

Table 3 shows the Gloss at 8° angle and ISO 2470 brightness value at gloss units (GU) for each lock versus its pattern.

**Table 3**

| Composition | Gloss at 8° vs pattern | ISO 2470 vs pattern |
|---|---|---|
| Example 1 | 10 | 19.69 |
| Comparative Composition 1 (Acai) | 1 | -2.68 |
| Comparative Compos. 2 (Murumuru) | 0 | 6.14 |
| Comparative Compos. 3 (Inca Inchi) | 6 | 7.99 |

Table 3 shows a synergistic effect of gloss for the composition of Example 1 containing Murumuru butter, Acai berry extract and Inca Inchi oil versus the comparative compositions containing only 1 of these 3 ingredients.

## Claims

1. A composition comprising a cosmetically effective amount of a Murumuru butter that ranges from 0.001% to 5% by weight, a cosmetically effective amount of an Acai berry extract that ranges from 0.0001% to 2% by weight and a cosmetically effective amount of an Inca Inchi oil that ranges from 0.005% to 15% by weight of the total composition.

2. The composition according to claim 1, comprising a cosmetically effective amount of Keratin Amino Acids.

3. The composition according to any of the previous claims, wherein the composition is a cosmetic composition

4. The composition according to any of the previous claims, wherein the composition is a hair cosmetic composition.

5. The composition according to any of the previous claims, wherein the composition is a hair care composition.

6. The composition according to any of the previous claims, wherein the composition is a shampoo, an oil, a serum, a rinse-off hair conditioner, a rinse-off hair mask, a leave-on hair conditioner or a leave-on hair mask.

7. The cosmetic composition according to any of the previous claims, wherein the composition comprises at least one additional cosmetic ingredient selected from the group of humectants, emollients, emulsifiers, surfactants, viscosity increasing ingredients, thickeners, binders, bulking agents, solvents, emulsion stabilizers, hair conditioners, natural extracts, antioxidants, pH adjusters, hair fixatives, film formers, UV filters, opacifying agents, fragrance ingredients, preservatives and cosmetic colorants.

8. A method of cosmetic treatment and/or care of the hair which comprises the topical application of a composition comprising a cosmetically effective amount of a Murumuru butter that ranges from 0.001% to 5% by weight, a cosmetically effective amount of an Acai berry extract that ranges from 0.0001% to 2% by weight and a cosmetically effective amount of an Inca Inchi oil that ranges from 0.005% to 15% by weight of the total composition.

9. The method according to claim 8, wherein the method is a cosmetic method of increasing the shine or gloss of the hair.

10. The method according to anyone of claims 8 to 9, wherein the composition is topically applied from about 20 seconds to about 45 minutes.

11. The method according to anyone of claims 8 to 9, wherein the composition is left-on the hair for at least 2 hours.

12. A process of cosmetic treatment and/or care of the hair which comprises the steps: A) topical application of a cleansing composition to the hair, B) rinsing the cleansing composition of step A), C) topical application of a conditioner composition to the hair, D) optionally, the conditioner composition of step C) is rinsed-off, wherein at least one of the cleansing composition of step A) or the conditioner composition of step C) is a composition comprising a cosmetically effective amount of a Murumuru butter that ranges from 0.001% to 5% by weight, a cosmetically effective amount of an Acai berry extract that ranges from 0.0001% to 2% by weight and a cosmetically effective amount of an Inca Inchi oil that ranges from 0.005% to 15% by weight of the total composition.

## Patentansprüche

1. Eine Zusammensetzung umfassend eine kosmetisch wirksame Menge einer Murumuru-Butter, die im Bereich von 0,001% bis 5 Gewichts % liegt, eine kosmetisch wirksame Menge eines Acai-Beerenextrakts, die im Bereich von 0,0001% bis 2 Gewichts % liegt, und eine kosmetisch wirksame Menge eines Inka-Inchi-Öls, die im Bereich von 0,005% bis 15 Gewichts % der Gesamtzusammensetzung liegt.

2. Die Zusammensetzung nach Anspruch 1, umfassend eine kosmetisch wirksame Menge Keratin Aminosäuren.

3. Die Zusammensetzung nach einem der vorhergehenden Ansprüche, wobei die Zusammensetzung eine kosmetische Zusammensetzung ist.

4. Die Zusammensetzung nach einem der vorhergehenden Ansprüche, wobei die Zusammensetzung eine haarkosmetische Zusammensetzung ist.

5. Die Zusammensetzung nach einem der vorhergehenden Ansprüche, wobei die Zusammensetzung eine Haarpflegezusammensetzung ist.

6. Die Zusammensetzung nach einem der vorhergehenden Ansprüche, wobei die Zusammensetzung ein Shampoo, ein Öl, ein Serum, eine ausspülbare Haarspülung, eine ausspülbare Haarmaske, eine Leave-on-Haarspülung oder eine Leave-on-Haarmaske ist.

7. Die kosmetische Zusammensetzung nach einem der vorhergehenden Ansprüche, wobei die Zusammensetzung mindestens einen zusätzlichen kosmetischen Bestandteil umfasst, ausgewählt aus der Gruppe von Feuchthaltemitteln, Weichmachern, Emulgatoren, Tensiden, viskositätserhöhenden Bestandteilen, Verdickungsmitteln, Bindemitteln, Füllstoffen, Lösungsmitteln, Emulsionsstabilisatoren, Haarkonditionierern, natürliche Extrakten, Antioxidantien, Substanzen zur Einstellung des pH-Wertes, Haarfixiermitteln, Filmbildnern, UV-Filtern, Trübungsmitteln, Duftstoffen, Konservierungsmitteln und kosmetischen Farbstoffen.

8. Eine Methode zur kosmetischen Behandlung und/oder Pflege des Haares, die umfasst, die topische Anwendung einer Zusammensetzung umfassend eine kosmetisch wirksame Menge einer Murumuru-Butter, die im Bereich von 0,001% bis 5 Gewichts % liegt, eine kosmetisch wirksame Menge eines Acai-Beerenextrakts, die im Bereich von 0,0001% bis 2 Gewichts % liegt, und eine kosmetisch wirksame Menge eines Inka-Inchi-Öls, die im Bereich von 0,005% bis 15 Gewichts % der Gesamtzusammensetzung liegt.

9. Die Methode nach Anspruch 8, wobei die Methode eine kosmetische Methode um den Glanz oder den Schein des Haares zu erhöhen ist.

10. Die Methode nach einem der Ansprüche 8 bis 9, wobei die Zusammensetzung von etwa 20 Sekunden bis etwa 45 Minuten topisch angewendet wird.

11. Die Methode nach einem der Ansprüche 8 bis 9, wobei die Zusammensetzung auf dem Haar mindestens 2 Stunden belassen wird.

12. Ein Verfahren zur kosmetischen Behandlung und/oder Pflege des Haares, das die Schritte umfasst: A) topische Anwendung einer Reinigungszusammensetzung zum Haar, B) Spülen der Reinigungszusammensetzung von Schritt A), C) topische Anwendung einer Konditioniererzusammensetzung zum Haar, D) gegebenenfalls wird die Konditioniererzusammensetzung von Schritt C) ausgespült, wobei mindestens eine der Reinigungszusammensetzung von Schritt A) oder die Konditioniererzusammensetzung von Schritt C) ist, eine Zusammensetzung umfassend eine kosmetisch wirksame Menge einer Murumuru-Butter, die im Bereich von 0,001% bis 5 Gewichts % liegt, eine kosmetisch wirksame Menge eines Acai-Beerenextrakts, die im Bereich von 0,0001% bis 2 Gewichts % liegt, und eine kosmetisch wirksame Menge eines Inka-Inchi-Öls, die im Bereich von 0,005% bis 15 Gewichts % der Gesamtzusammensetzung liegt.

## Revendications

1. Composition comprenant une quantité cosmétiquement efficace d'un beurre de Murumuru qui varie entre 0,001% et 5% en poids, une quantité cosmétiquement efficace d'un extrait de baies d'Açaï qui varie entre 0,0001% et 2% en poids et une quantité cosmétiquement efficace d'une huile d'Inca Inchi qui varie entre 0,005% et 15% en poids par rapport au poids total de la composition.

2. Composition selon la revendication 1, comprenant une quantité cosmétiquement efficace d'acides aminés de kératine.

3. Composition selon l'une quelconque des revendications précédentes, ladite composition étant une composition cosmétique.

4. Composition selon l'une quelconque des revendications précédentes, ladite composition étant une composition cosmétique capillaire.

5. Composition selon l'une quelconque des revendications précédentes, ladite composition étant une composition de soin capillaire.

6. Composition selon l'une quelconque des revendications précédentes, ladite composition étant un shampooing, une huile, un sérum, un après-shampoing à rincer, un masque capillaire à rincer, un après-shampoing sans rinçage ou un masque capillaire sans rinçage.

7. Composition cosmétique selon l'une quelconque des revendications précédentes, dans laquelle la composition comprend au moins un ingrédient cosmétique supplémentaire choisi parmi le groupe des humectants, émollients, émulsifiants, tensioactifs, ingrédients augmentant la viscosité, épaississants, liants, agents de charge, solvants, stabilisateurs d'émulsion, conditionneurs capillaires, extraits naturels, antioxydants, ajusteurs de pH, fixateurs capillaires, agents filmogènes, filtres UV, agents opacifiants, ingrédients de parfum, conservateurs et colorants cosmétiques.

8. Méthode de traitement cosmétique et/ou de soin des cheveux qui comprend l'application topique d'une composition comprenant une quantité cosmétiquement efficace d'un beurre de Murumuru qui varie entre 0,001% et 5% en poids, une quantité cosmétiquement efficace d'un extrait de baies d'Açaï qui varie entre 0,0001% et 2% en poids et une quantité cosmétiquement efficace d'une huile d'Inca Inchi qui varie entre 0,005% et 15% en poids par rapport au poids total de la composition.

9. Méthode selon la revendication 8, ladite méthode étant une méthode cosmétique pour augmenter l'éclat ou la brillance des cheveux.

10. Méthode selon l'une quelconque des revendications 8 ou 9, dans laquelle la composition est appliquée topiquement pendant une durée d'environ 20 secondes à environ 45 minutes.

11. Méthode selon l'une quelconque des revendications 8 ou 9, dans laquelle la composition est laissée sur les cheveux pendant au moins 2 heures.

12. Procédé de traitement cosmétique et/ou de soin des cheveux qui comprend les étapes: A) application topique d'une composition nettoyante sur les cheveux, B) rinçage de la composition nettoyante de l'étape A), C) application topique d'une composition de conditionneur sur les cheveux, D) de façon optionnelle, la composition de conditionneur de l'étape C) est rincée, dans lequel au moins une de la composition de nettoyage de l'étape A) ou de la composition de conditionneur de l'étape C) est une composition comprenant une quantité cosmétiquement efficace d'un beurre de Murumuru qui varie entre 0,001% et 5% en poids, une quantité cosmétiquement efficace d'un extrait de baies d'Açaï qui varie entre 0,0001% et 2% en poids et une quantité cosmétiquement efficace d'une huile d'Inca Inchi qui varie entre 0,005% et 15% en poids par rapport au poids total de la composition.
